# FASCICULE DE BREVET EUROPEEN

(11) **EP 3 167 177 B1**
(45) Date de publication et mention de la délivrance du brevet: **09.06.2021**
(21) Numéro de dépôt: 15759502.6
(22) Date de dépôt: 09.07.2015
(51) Int. Cl.: F02F 1/38

(54) **CULASSE, ELEMENT ET SEMELLE DE MOTEUR A PISTON**
ZYLINDERKOPF, ELEMENT UND FLANSCH EINES KOLBENMOTORS
CYLINDER HEAD, ELEMENT AND FLANGE OF A PISTON ENGINE

(30) Priorité: 09.07.2014 FR 1456618
(43) Date de publication de la demande: 17.05.2017
(73) Titulaire: Mecachrome France, 37403 Amboise Cedex (FR)
(72) Inventeur: HUBERT, Pierre-Julien, F-91430 Igny (FR); BOUDY, Jean-Pierre, F-91570 Bievres (FR); VERMEIREN, Sébastien, F-78800 Versailles (FR)
(74) Mandataire: Benech, Frédéric
(86) Numéro de dépôt international: PCT/FR2015/051900
(87) Numéro de publication internationale: WO 2016/005704

(56) Documents cités:
- WO-A1-03/062621
- DE-A1- 2 514 592
- US-A- 1 479 129
- US-A- 4 018 195
- US-A- 4 112 906

## Description

La présente invention concerne une culasse de moteur à piston s'étendant longitudinalement du type comprenant un corps de culasse et une semelle en au moins un élément, la semelle étant à interposer entre une chambre de combustion et le corps de culasse, comportant une face supérieure en vis-à-vis du corps de culasse et une face inférieure opposée à disposer face à la chambre de combustion et étant rapportée sur le corps de culasse.

Elle trouve une application particulièrement importante bien que non exclusive dans le domaine des moteurs compacts, de masse réduite, à haute densité de puissance (rapport entre la puissance mécanique fournie par le moteur et le volume total correspondant au volume maximal balayé par le piston), à très haut rendement thermodynamique notamment pour objet volant ou voiture de course.

Améliorer le rendement du cycle thermodynamique d'un moteur à piston, quel que soit le cycle choisi (Carnot, Stirling, Diesel...), est une préoccupation constante du constructeur de moteurs.

Le rendement des moteurs dépend de la compression et de l'apport d'énergie dans la chambre de combustion, et plus particulièrement des valeurs maximales respectives des températures et des pressions qui peuvent y être atteintes.

Il est connu d'augmenter la pression par une suralimentation en comburant (air) dans la chambre de combustion.

Néanmoins, cette technique implique d'opérer un arbitrage entre pression dans la chambre et poids du moteur.

En effet, pour tenir à des pressions élevées et à la température de combustion du carburant, il est nécessaire de choisir des matériaux mécaniquement résistants à ces conditions de pression et température et/ou d'adapter le dimensionnement des pièces au matériau choisi.

Or ce type de matériaux, tel que par exemple l'acier ou la fonte, sont lourds (masse volumique supérieure à 5000 kg/m³).

Pour pallier à cet inconvénient, il a été proposé un moteur comprenant un bloc moteur en matériau résistant et une culasse en matériau plus léger.

Mais, dans ce cas, la culasse possède une face en vis-à-vis de la chambre de combustion, dite face feu, en matériaux peu résistants, ce qui limite les possibilités du moteur.

On connaît également (EP 0 262 240) une culasse en deux parties soudées, en matériaux différents.

Une telle culasse présente cependant toujours des inconvénients.

En effet, elle ne permet toujours pas de fonctionner dans des gammes de températures et de pressions élevées, et ce en particulier si on prévoit une épaisseur de semelle faible pour limiter le poids.

Le montage n'est pas simple et/ou réversible, et nécessite une opération de soudure entre deux matériaux différents, toujours complexe et coûteuse.

Enfin, les pièces doivent être de géométrie parfaite car le moindre défaut de côtes est générateur d'une importante chute de performance.

On connaît aussi (WO 03/062621) un dispositif composé de deux matériaux différents mais avec une mauvaise résistance aux vibrations et mettant en œuvre une couche adhésive entre eux.

Le document (DE 25 14 592) décrit quant à lui une culasse en deux parties, monocylindrique avec chambre de refroidissement présentant des inconvénients de corrosion.

La présente invention vise à pallier ces inconvénients et fournir une culasse répondant mieux que ceux antérieurement connus aux exigences de la pratique, notamment en ce qu'elle permet d'atteindre des rendements supérieurs aux moteurs de l'art antérieur sans que cela ne soit au détriment du poids.

Ainsi l'invention permet d'atteindre des pressions dans la chambre de combustion (pression cylindre) importantes avec des hauts de culasse par exemple en alliage léger ou, pour une pression de compression maximale donnée, de réduire le poids par rapport à une culasse en alliage lourd.

En fait, il va par exemple être possible, avec l'invention, et alors que les culasses de l'art antérieur fonctionnent classiquement à des pressions de 180 à 200 bars et des températures de chambre de combustion inférieures à 200°C, d'obtenir des pressions supérieures à 250 ou même 300 bars, et des températures supérieures à 250°C, d'où les excellents rendements observés.

L'invention permet par ailleurs un assemblage plus simple et rapide de la culasse sur le moteur, de façon moins onéreuse. Elle permet également un assemblage assurant une meilleure planéité de la face inférieure de la semelle, ou des éléments de semelle entre eux.

Enfin, elle permet également un remplacement et/ou un recyclage des éléments de la culasse.

Dans ce but, l'invention propose notamment une culasse de moteur à piston s'étendant longitudinalement comprenant un corps de culasse et une semelle en au moins un élément, la semelle étant à interposer entre une chambre de combustion et le corps de culasse, comportant une face supérieure en vis-à-vis du corps de culasse et une face inférieure opposée à disposer face à la chambre de combustion et étant rapportée sur le corps de culasse, la semelle et le corps sont assemblés par des vis de fixation de la semelle sur la culasse situées vers la périphérie de la semelle et du corps et par au moins une pièce de fixation de la culasse sur la semelle comprenant une extrémité filetée de fixation dans un orifice correspondant de la semelle, ledit orifice étant situé plus proche du centre de la semelle que les vis de fixation, caractérisée en ce que le corps de culasse est en un matériau d'une masse volumique et d'une dureté plus faibles que celles du matériau constitutif de la semelle et en ce que l'effort de serrage de la ou des pièces de fixation est supérieur ou égal à deux fois l'effort de serrage des vis de fixation de la semelle.

Par dureté on entend la résistance qu'oppose une surface d'un échantillon de matière considéré à la pénétration d'un poinçon, par exemple une bille en acier trempé (dureté Brinell suivant la norme EN ISO 6506 révisée en 2005) ou une pyramide en diamant (dureté Vickers suivant la norme EN ISO 6507 révisée en 2005).

Dans un mode de réalisation avantageux de l'invention, le critère de dureté est remplacé par la limite d'endurance.

Par limite d'endurance, σ_{D}(N) on entend la limite en fatigue pour un type de sollicitations (avantageusement traction, mais également flexion, torsion) et pour une contrainte moyenne σₘ telle que définie dans la norme NF A 03-400 (mise à jour Août 1983) pour un nombre de cycles N (par exemple 10⁶) de sollicitations appliquées à une éprouvette de forme déterminée (par exemple de section cylindrique de diamètre 3mm, de longueur 1cm) et d'état de surface déterminé, à une température et/ou dans une plage de températures donnée (par exemple entre 100°C et 400°C, par exemple 350°C).

Pour effectuer la mesure on sollicite l'éprouvette jusqu'à ce qu'elle ait atteint le critère de fin d'essai (fin du nombre de cycles ou rupture de l'éprouvette).

La valeur de la limite d'endurance vers laquelle tend l'amplitude de contrainte après N cycles, dépendant de la géométrie de l'éprouvette et de la température, les comparaisons entre limites d'endurance entre matériaux devront donc être bien entendu réalisées dans les mêmes conditions d'éprouvette et de température.

De façon générale, la limite d'endurance s'écrit σ_{D}(N) = σₘ ± σₐ avec σₐ amplitude de la contrainte correspondant à la limite d'endurance pour une valeur particulière de la contrainte moyenne σₘ.

Par centre on entend l'isobarycentre de la face inférieure de la semelle.

Par vers la périphérie, il faut entendre par exemple la zone de la semelle s'étendant depuis les bords extérieurs de celle-ci jusqu'à une ligne homothétique du bord située à quelques millimètres de celle-ci, par exemple 1 à 2 cm.

Par ailleurs, l'effort de serrage de la ou des pièces de fixation est par exemple supérieur ou égal à trois fois, avantageusement quatre fois, l'effort de serrage des vis de fixation de la semelle.

La fixation par vis aurait pu paraître fragilisante pour l'homme du métier, compte tenu des contraintes mécaniques générées par le moteur, engendrant par ailleurs une déformation de la semelle (effet peau de tambour). L'invention permet de limiter suffisamment et/ou éliminer ces déformations, préjudicielles à la tenue mécanique, en ajoutant au moins un point de serrage, avantageusement central ou vers le milieu, pour éviter le décollement de la semelle, ou de l'élément de semelle si elle est en plusieurs éléments, dans la zone centrale, ledit point de serrage étant situé à l'intérieur du joint fermé d'étanchéité entre culasse et semelle, s'il y a lieu.

Comme on l'a vu et pour assurer un assemblage correct de la culasse et des éléments de semelle, le serrage entre eux se fait par des vis de fixation périphérique et par au moins une pièce de fixation, de position plus centrale par rapport aux vis, afin également de répartir correctement les pressions de contact.

La semelle en au moins un élément, une fois montée sur le corps de culasse, a sa face inférieure (accessible et de l'autre côté du montage) qui est usinée pour assurer notamment sa planéité avant sa fixation définitive sur le bloc moteur.

En effet, celle-ci est prise en sandwich entre le bloc moteur et le corps de culasse et possède différents appuis (joint de culasse et tablature du bloc moteur). Elle va de ce fait légèrement se déformer en pression, et en particulier, les portées de sièges de soupape vont s'incliner, au détriment de la performance et de l'étanchéité du moteur.

Le procédé d'assemblage permet ici de rectifier les déformations de la semelle lors de l'assemblage sur le bloc moteur et donc d'assurer une meilleure conformité géométrique en fonctionnement.

Après plusieurs itérations par exemple par méthodologie essais/erreurs, des valeurs d'efforts de serrage de la pièce de fixation sont établies qui permettent d'obtenir une déformation de la semelle, et plus particulièrement de sa face inférieure, avant son usinage, proche et/ou identique avec celle de sa déformation réelle (lorsque l'on applique une valeur nominale d'effort de serrage à la pièce de fixation une fois la semelle montée sur le bloc moteur), les autres éléments de fixations étant serrés à leur valeur nominale (vis de fixation, goujon, ...).

La pré-déformation ainsi obtenue avant l'usinage permet d'anticiper les déformations nominales de fonctionnement et de les compenser, cela limite ainsi voir supprime, les déformations résiduelles dues aux différents serrages par usinage.

Dans des modes de réalisation avantageux, on a de plus et/ou en outre recours à l'une et/ou à l'autre des dispositions suivantes :
- le corps de culasse est en un matériau d'une conductivité thermique plus forte que celle du matériau constitutif de la semelle ;
- le corps de culasse est en aluminium, en alliage à base d'aluminium, en magnésium, ou en alliage à base de magnésium et la semelle est en acier ou en alliage à base de nickel ;
- l'orifice est situé ou sensiblement situé dans un plan longitudinal centré et orthogonal par rapport à la semelle. Par sensiblement situé on entend autour de la ligne (de symétrie) longitudinale centrale de la culasse, par exemple à une distance inférieure à +/- 5 cm de la ligne;
- la semelle comprend au moins deux éléments juxtaposés identiques coplanaires comprenant chacun quatre vis de fixation (par exemple à chaque angle) et un orifice. Un nombre différent de vis périphériques par élément peut également être prévu ;
- l'orifice est centré par rapport à la semelle et/ou l'élément de semelle ;
- l'orifice est traversant de la semelle ;
- la pièce de fixation comprend un fourreau agencé pour recevoir individuellement un premier dispositif fonctionnel utile au fonctionnement du moteur et devant interagir avec la chambre de combustion. Ceci confère à l'ensemble une excellente rigidité axiale, tout en permettant un accès par le biais du fourreau à l'intérieur de la chambre de combustion ;
- la ou les pièces de fixation de la culasse passent à travers des orifices qui traversent le corps de part en part, prennent appui sur le corps et sont vissées dans la semelle, et les vis de fixation de la semelle prennent appui sur la semelle et sont vissées dans le corps ;
- la semelle s'étendant globalement dans un plan, comprend un circuit interne de refroidissement par circulation d'un fluide caloporteur, ledit circuit comprenant au moins une arrivée de fluide, un lit de rivière de circulation du fluide dans son épaisseur et plusieurs canaux d'évacuation du fluide dans le corps de la culasse, les canaux formant un angle supérieur à 20° par rapport au plan de la semelle en passant autour des conduits d'échappement des gaz du corps de culasse.

Par lit de rivière il est entendu ici aussi bien un canal affleurant sur une face de la semelle et donc ouvert sur cette face comme par exemple un lit formé par usinage de la face supérieure de la semelle. On entend également par lit, un canal interne entièrement compris dans l'épaisseur de la semelle. C'est-à-dire en reprenant l'exemple précédent la face supérieur est pleine et éventuellement plane, une telle semelle pouvant alors être obtenue par exemple par fonderie.

Grâce à une telle disposition, on réalise un refroidissement local avec très peu d'eau (1/2 litre d'eau pour 2 à 3 litres d'eau avec une culasse traditionnelle) et ce de façon rapide.

L'eau monte du bloc, passe dans la semelle avant de refroidir le corps de culasse grâce aux canaux d'évacuation formant des rideaux de refroidissement autour des conduits d'échappement très chauds ;
- le lit de rivière, s'étendant suivant un parcours, est d'une largeur moyenne 1 inférieure à 20% de la dimension transversale ou largeur de la semelle et comprend le long dudit parcours au moins un étranglement allongé sur au moins 2,5% de la longueur du parcours et la largeur moyenne de l'étranglement parallèlement au plan de la semelle est inférieure à 10% de la largeur de la semelle. L'étranglement permet l'accélération du fluide de refroidissement là où l'on souhaite la meilleure efficacité de refroidissement.

La dimension transversale est la dimension perpendiculaire à la direction longitudinale de la semelle ;
- le circuit intérieur de la semelle présente des perçages intersièges de soupape. Cette disposition permet de refroidir les parties intersièges de soupape, toujours difficile à atteindre ;
- la semelle comporte au moins un siège de soupape d'une seule pièce avec la semelle. Les sièges de soupapes peuvent donc être rapporté ou non. Le siège peut ainsi être fabriqué en un matériau plus spécifique et/ou être changé, ce qui évite de remplacer la totalité de la semelle ou de l'élément ;
- la culasse comprend au moins un joint d'étanchéité entre le corps et la semelle ;
- le corps de culasse comprenant des conduits d'admission et/ou d'échappement, les conduits d'échappement sont munis d'un manchon en un matériau ayant une conductivité thermique très faible, ledit manchon étant bloqué en position vis-à-vis du conduit d'échappement par des portions d'extrémités à l'écart desquelles un espace existe entre ledit manchon et la portion de conduit d'échappement en vis-à-vis, pour limiter les échanges thermiques.

Par conductivité thermique très faible on entend plus précisément ici une conductivité thermique ρ inférieure à 70 W.m⁻¹.K⁻¹ par exemple inférieure à 40 W.m⁻¹.K⁻¹, par exemple 20 W.m⁻¹.K⁻¹ comme celle du titane;
- la semelle comporte une ouverture de passage d'un second dispositif fonctionnel utile au fonctionnement du moteur et devant interagir avec la chambre de combustion.

Le second dispositif peut être de toute sorte et être compris ou non dans un fourreau permettant une bonne adaptation géométrique.

Est décrit un élément d'une semelle pour culasse de moteur à piston, l'élément s'étendant globalement dans un plan, comportant une face supérieure et une face inférieure opposée et étant propre à être interposé comme interface entre une chambre de combustion du moteur et un corps de la culasse, la face supérieure présentant un circuit interne de refroidissement par circulation d'un fluide caloporteur, cet élément étant agencé pour être disposé de façon coplanaire avec d'autres éléments identiques de la semelle,
et présentant :
des premiers orifices de passage de vis de fixation de l'élément avec le corps de culasse, situés vers la périphérie de l'élément et
au moins un second orifice de vissage d'une pièce de fixation du corps de culasse avec ledit élément situé plus près ou proche du centre de la semelle que les premiers orifices de fixation.

Est aussi décrite une semelle pour culasse de moteur à piston, la semelle étant allongée dans une direction, s'étendant globalement dans un plan comportant une face supérieure et une face inférieure opposée, et étant propre à être interposée comme élément d'interface entre une chambre de combustion du moteur et un corps de la culasse, dans laquelle la face supérieure présente au moins un circuit interne de refroidissement par circulation d'un fluide caloporteur, cette semelle présentant :
des premiers orifices de passage de vis de fixation de la semelle avec le corps de culasse, s'échelonnant globalement en périphérie et le long de la direction d'allongement de la semelle, et
au moins un second orifice de vissage d'au moins une pièce de fixation du corps de culasse avec ledit élément situés plus près du centre de la semelle que les premiers orifices de fixation.

Dans d'autres modes de réalisation avantageux de la semelle ou de l'élément de semelle, le circuit interne de refroidissement est compris dans l'épaisseur de la semelle et la face supérieure est plane ou sensiblement plane. Dans d'autres modes de réalisation également avantageux c'est la face supérieure qui présente au moins un circuit interne de refroidissement [lit de rivière (à ciel ouvert)].

L'invention propose de plus un ensemble moteur comprenant une culasse du type décrit ci-dessus,
caractérisé en ce que il comporte des injecteurs de carburant des bougies ou des dispositifs d'allumage, montés dans les fourreaux en tant que pièces fonctionnelles.

Par dispositif d'allumage, on entend notamment les dispositifs d'allumage de la combustion de carburant/comburant dans un moteur à allumage contrôlé.

L'invention sera mieux comprise à la lecture de la description qui suit d'un mode de réalisation donné ci-après à titre d'exemple non limitatif. La description se réfère aux dessins qui l'accompagnent dans lesquels :
La figure 1 est une vue de dessous de la face inférieure d'une culasse selon un mode réalisation de l'invention, à monter sur un bloc moteur.
La figure 2 montre une vue en perspective de dessus d'un élément d'une semelle de la culasse de la figure 1.
La figure 3 est une vue en perspective éclatée d'une culasse selon le mode de réalisation des figures 1 et 2.
La figure 4 est une vue en coupe selon IV-IV de la figure 1.
La figure 5 est une vue en perspective éclatée d'un fourreau d'injecteur et de ses pièces supports, utilisable comme pièce de fixation avec l'élément de semelle de la figure 2.
La figure 6 est une vue partielle en coupe selon VI-VI de la figure 1 montrant l'installation d'un capteur de pression de la chambre de combustion.
La figure 7 montre de manière schématique, en perspective de dessous et en volume, une partie du circuit de circulation du fluide de refroidissement de la culasse et de la semelle décrite en références aux figures 1 à 4, (passant notamment autour des conduits d'alimentation et d'échappement d'une chambre).
La figure 8 donne une autre vue, en perspective de dessus et en volume, de la figure 7 illustrant les points chauds.
Les figures 9 et 10 présentent deux vues en coupe selon IX-IX et X-X d'un ensemble comprenant la culasse de moteur de la figure 1 respectivement au niveau de l'injecteur, et au niveau des soupapes.

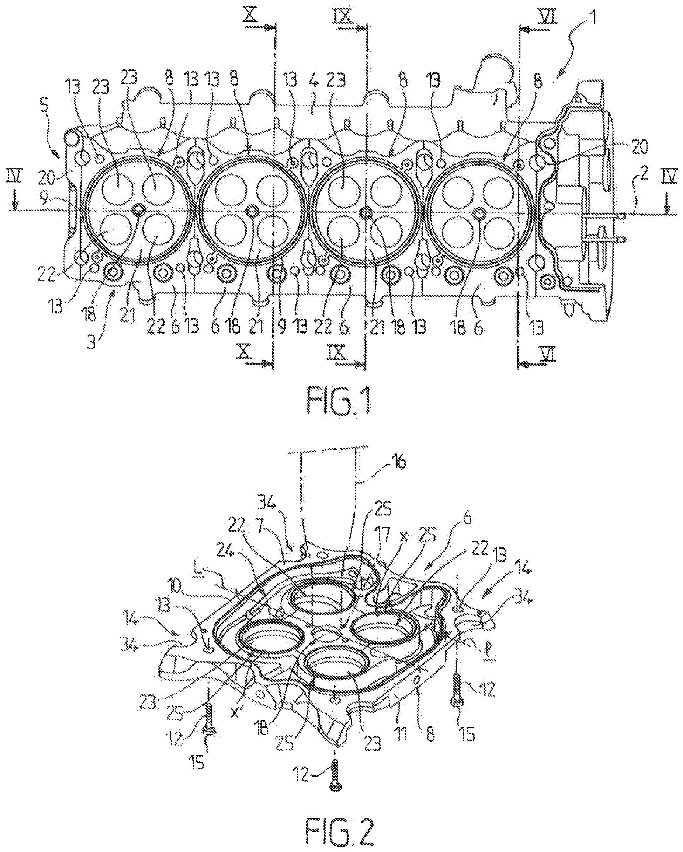

La figure 1 montre en vue de dessous une culasse 1 de moteur à piston (ici quatre pistons) s'étendant longitudinalement le long d'un axe 2, vue du côté de la face feu 3.

Elle comprend un corps 4 de culasse (ou haut de culasse) et une semelle 5 formée de quatre éléments 6 identiques correspondant à chacun des pistons.

En référence également à la figure 2 ces éléments 6 comportent une face supérieure 7 destinée à être en vis à vis du corps ou haut de culasse 4 et une face inférieure 8, par exemple plane, munie d'un joint métallique circulaire 9 en vis à vis de la périphérie supérieure de chacune des quatre chambres de combustion des pistons concernés.

Le corps de culasse et la semelle et les quatre éléments 6 sont en matériaux métalliques.

Le corps de culasse étant d'un premier matériau d'une masse volumique ρ₁ et d'une dureté par exemple dureté Vickers HV1, et/ou d'une limite en fatigue σ_{D1}(N), la semelle et plus particulièrement ces quatre éléments 6 sont quant à eux d'une masse volumique ρ₂ et d'une dureté HV2 et/ou d'une limite en fatigue σ_{D2}(N).

Dans le mode de réalisation plus particulièrement décrit ici le corps de culasse est également d'un premier matériau d'une conductivité thermique λ₁, d'un module d'Young E₁, et d'une limite d'élasticité Re1, la semelle et plus particulièrement ces quatre éléments 6 sont quant à eux d'une conductivité thermique λ₂, d'un module d'Young E₂ et d'une limite d'élasticité Re2.

La masse volumique ρ₁ et la dureté HV1 et/ou la limite en fatigue σ_{D1}(N) sont quant à elles plus faibles, c'est à dire respectivement inférieur à la masse volumique ρ₂ et à la dureté HV2 et/ou la limite en fatigue σ_{D2}(N).

Dans le mode de réalisation plus particulièrement décrit ici la conductivité thermique λ₁ du corps de culasse est plus fort, c'est-à-dire supérieure, à la conductivité thermique λ₂ de la semelle, et la limite d'élasticité Re1 est plus faible que Re2.

Le matériau de la culasse est par exemple en aluminium ou en alliage d'aluminium et le matériau des semelles ou des éléments de semelles est en acier par exemple en acier 316L.

Selon le mode de réalisation de l'invention plus particulièrement décrit ici les éléments de semelles sont en forme de plaque 10 de forme généralement parallélépipédique, par exemple sensiblement carrée dont la périphérie 11 est conçue et agencée, pour permettre les adaptations nécessaires avec le corps de culasse, adaptations notamment liées aux orifices d'admission et d'échappement et aux goujons de fixation de la culasse sur le bloc moteur, et ce de façon connue en elle-même.

Chacun des éléments est assemblé au corps par l'intermédiaire de quatre vis 12 de fixation de l'élément de semelle sur la culasse, placées vers la périphérie 11 desdits éléments de semelle.

Plus précisément chaque vis est formée d'une tête d'appui plate, d'un corps cylindrique lisse allongé terminé par une partie filetée agencée pour dépasser de l'autre côté de la semelle lorsqu'elle est introduite dans un trou traversant 13 correspondant de la plaque.

Celle-ci comporte en effet quatre trous 13 situés aux quatre coins 14 de l'élément de semelle de façon traversante.

Les têtes d'appui 15 viennent en appui sur la face externe 8 de l'élément de semelle, par exemple dans des encoches dans lesquelles elles viennent s'encastrer complètement pour ne pas dépasser du plan de la face 3.

La partie filetée des vis 12 vient quant à elle coopérer avec des orifices taraudés en vis à vis sur la face externe inférieure plane F du haut de culasse.

Chaque élément de semelle est également fixé sur le haut de culasse par l'intermédiaire d'une pièce de fixation 16 symbolisée en trait mixte sur la figure 2, traversant entièrement le haut de culasse, centré par rapport à l'élément de semelle correspondant et comprenant une extrémité 17, filetée, de fixation dans un orifice taraudé 18, ici, traversant de la semelle mais qui peut également être borgne. L'orifice est donc situé au centre ou sensiblement au centre de la semelle par rapport aux vis de fixation.

De façon à avoir des éléments de semelles 6 identiques et compte tenu de la configuration de la face inférieure F du haut de culasse, des éléments d'extrémité 20 sont prévus, situés de part et d'autre et forment des sortes de lamelles de même épaisseur que la semelle. Ils sont fixés également par l'intermédiaire de vis traversantes sur la face externe du haut de culasse. Les éléments d'extrémité 20 servent de soutien et assurent la répartition de l'effort d'assemblage du corps de culasse sur le bloc moteur.

Ils sont donc de même épaisseur que les éléments 6 de semelles de forme correspondante au profit de la zone du corps de culasse à l'extrémité longitudinal de celui-ci. Ils sont donc fixés par des vis de fixation semblables à celles utilisées pour les éléments de semelles.

En référence à la figure 1, il apparaît que à chaque élément de semelle 6 associé à une chambre de combustion correspondante (disque circulaire 21), il est prévu quatre orifices cylindriques traversant, à savoir deux orifices d'admission 22 et deux orifices d'échappement 23, associés aux soupapes correspondantes de façon connue en elle-même.

Chaque élément de semelle et la partie correspondante du haut de culasse comprend un circuit interne de refroidissement. Plus précisément, la face supérieure 7 de l'élément 6 comprend un lit de rivière 24 de circulation dans son épaisseur, qui s'étend suivant un parcours agencé pour permettre le refroidissement optimisé de la plaque qui compte tenu de son faible coefficient λ, à tendance à fortement chauffer. Pour ce faire on prévoit par calcul et de façon connue par l'homme du métier technicien une largeur moyenne de rivière 1 de dimension suffisante mais propre à être inférieure à 15% de la dimension de la dimension transversale (ou largeur) L de la semelle.

Une telle disposition permet de ne pas fragiliser la semelle compte tenu des conditions de pression par ailleurs mises en œuvre.

Le circuit interne comprend de plus le long du parcours, plusieurs étranglements 25 disposés aux points les plus chauds de la plaque de façon à accélérer le début de refroidissement à ces emplacements.

Avantageusement la largeur cumulée de ces rétrécissements correspondent au total à un pourcentage de 20% de la longueur du parcours. Les étranglements sont par exemple d'une largeur x = 3mm parallèlement au plan de la semelle, inférieure à 5% de la largeur de la semelle.

Les dimensionnements x, x'... de ces rétrécissements sont calculés par l'homme du métier en tenant compte des paramètres de contraintes mécaniques, de perte de charge et de températures attendues dans ladite culasse en absence de refroidissement.

Dans la suite de la description on utilisera les mêmes numéros de références pour désigner les mêmes éléments.

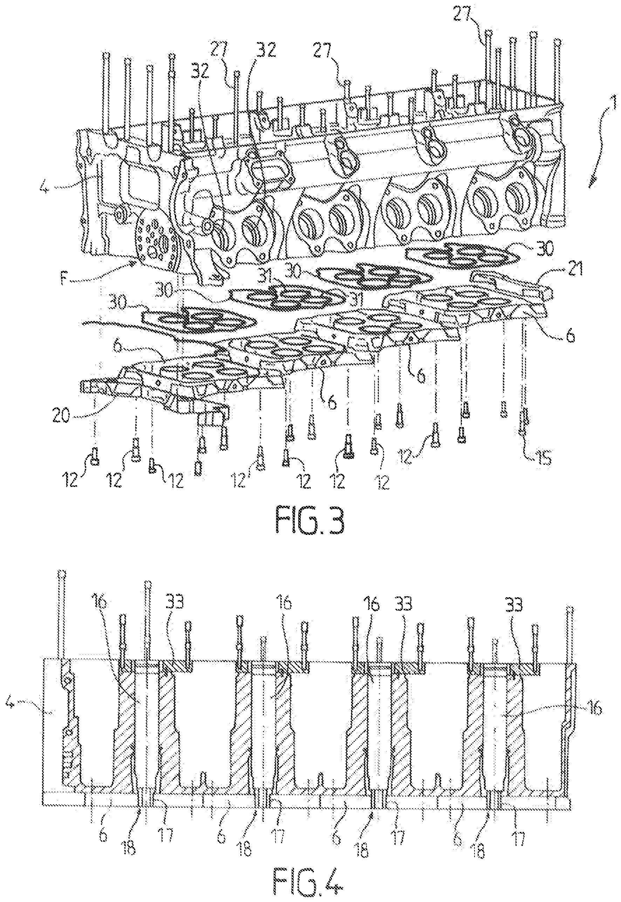

La figure 3 montre la culasse 1 en perspective, de la figure 1. Elle comprend le haut de culasse 4 et les éléments de semelles 6 du type décrit en référence à la figure 2. Ceux-ci sont donc fixés sur le haut de culasse par l'intermédiaire des vis 12 comme décrit ci-avant, vis traversantes de chaque élément pour venir coopérer avec un trou taraudé existant en vis à vis, trou réalisé dans la face inférieure F du haut de culasse.

Des joints d'étanchéïté 30 et 31, de type joint torique, de forme adéquate, sont prévus sur les faces supérieures 7 des éléments 6, respectivement sur le pourtour de l'élément de semelle, vers l'extérieur par rapport au circuit interne de refroidissement d'une part, et au niveau des ouvertures d'échappement et d'admission d'autre part.

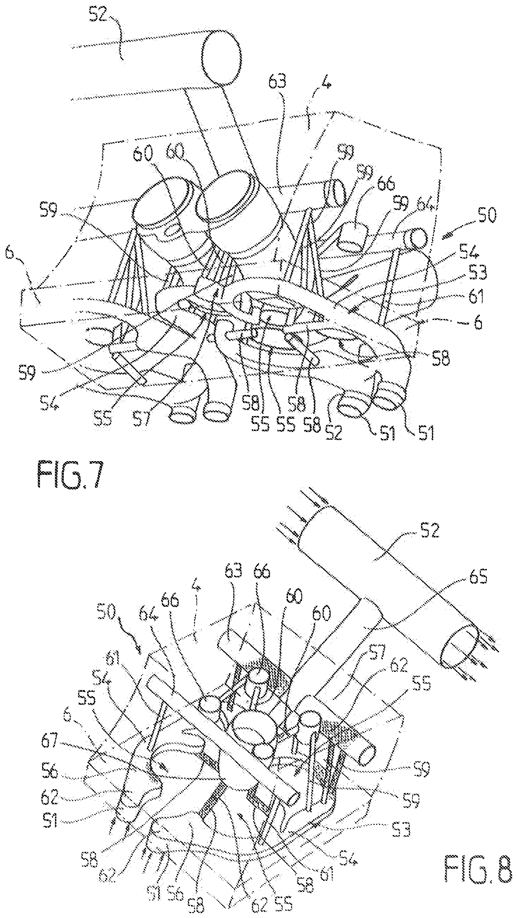

Le haut de culasse est par exemple un alliage d'aluminium et est quant à lui constitué pour l'essentiel de façon connue en elle-même, sauf en ce qui concerne le circuit de refroidissement qui est plus spécifique et dont la description va être complétée ci-après en référence aux figures 7 et 8.

De façon connue le haut de culasse comprend des conduits 32 d'admission en biais et des conduits d'échappement situés de l'autre côté de la culasse, par exemple munis comme on le verra par la suite d'un manchon isolant en température.

On a représenté sur la figure 4 une coupe selon IV IV de la figure 1, montrant les pièces de fixation 16 qui sont constituées par des fourreaux agencés pour recevoir les injecteurs dans le cas du moteur diesel plus particulièrement décrit ici.

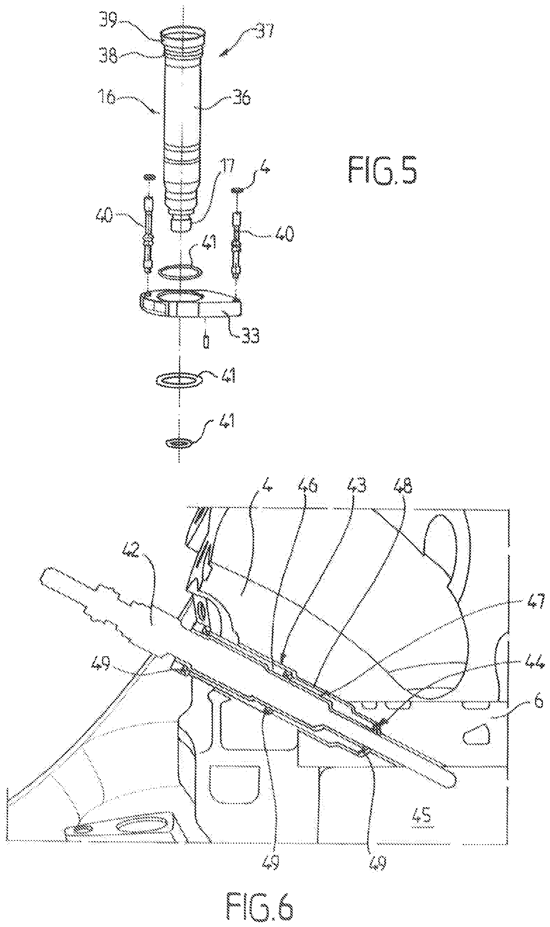

Ces fourreaux sont en appui ou vissés d'un côté sur une couronne 33 venant en appui sur le haut de culasse comme cela sera plus précisément décrit en référence à la figure 5.

La fixation de l'autre côté de ces fourreaux, propres à recevoir les injecteurs d'injection du carburant au centre des chambres de combustion via l'orifice central 18 dans les plaques formées par les semelles 6, permet de reprendre les efforts liés à la pression dans les chambres et d'éviter ainsi le bombement de chaque élément de semelle et d'obtenir des performances bien meilleures que dans l'art antérieur (à poids de culasse égal).

Sur le mode de réalisation décrit l'élément de semelle 6 comporte quatre encoches 34 semi-cylindriques, réparties symétriquement par rapport au plan longitudinal passant par l'axe 2. Les encoches 34 de deux éléments 6 respectifs se font face pour former un passage pour les goujons 27, de fixation du corps 4 de culasse sur le bloc moteur (non représenté) au travers des éléments de semelles 6.

Ceux-ci sont d'une épaisseur e calculée pour résister aux contraintes de température et de pression des chambres de combustion, tout en permettant un refroidissement efficace et optimisé par le circuit interne de refroidissement. L'épaisseur e est ainsi et par exemple comprise entre 2 cm et 5 cm, par exemple 3 cm.

La figure 5 montre la pièce 16 formée par un fourreau d'injecteur et ses éléments de montage 35 comprenant la couronne 33. Le fourreau est formé d'un corps cylindrique 36 par exemple en alliage métallique comme le matériau connu de l'homme du métier sous la référence 40CDV20, agencé pour coopérer à frottement doux à l'intérieur d'un alésage correspondant du haut de culasse. Le corps 36 comprend une extrémité supérieure 37 présentant une partie bilatérale 38 et une collerette 39 partie en excroissance d'appui sur la couronne amovible 33, cette dernière étant agencée pour elle-même venir en appui dans un évidement ménagé à cet effet dans la face supérieure du haut de culasse (non représenté sur la figure 5).

La couronne 33 est par exemple formée par une plaque percée d'un orifice de passage à frottement doux du corps excentré par rapport à la plaque qui est donc de forme indexable avec la culasse. La plaque est fixée sur cette dernière par le biais de vis 40.

Le corps 36 comprend l'extrémité inférieure 17 de la pièce de fixation 16, filetée et agencée pour se visser dans l'orifice transversal 18 correspondant comme décrit ci-avant.

Des joints toriques d'étanchéité 41 sont prévus de façon connue en elle-même et aux endroits nécessaires tel qu'anticipé par l'homme du métier.

La figure 6 montre en vue partielle et en coupe l'installation d'un capteur 42 de pression, ou de tout autre dispositif fonctionnel tel que par exemple capteur de température, injecteur, bougie, ou dispositif de prélèvement de gaz. Le capteur 42 est installé par le biais d'un canal d'accès 43 sensiblement cylindrique percé dans le haut de culasse 4 et prolongé par un orifice 44 oblique réalisé dans l'élément de semelle 6 de séparation avec la chambre de combustion 45.

Le canal d'accès 43 et l'orifice oblique 44 sont agencés pour aménager un espace vide 46 entre la surface externe 47 du capteur 42 et sa paroi interne 48, de manière à constituer une isolation thermique entre le capteur, la culasse et la semelle.

Des joints d'étanchéité 49 sont par ailleurs prévus de façon connue en elle-même.

On a représenté sur les figures 7 et 8 le circuit interne 50 de refroidissement des éléments de semelle 6 et de haut de culasse 4 (schématisés en trait mixte), mis en œuvre selon le mode de réalisation de l'invention plus particulièrement décrit ici.

Le circuit 50 comprend deux arrivées d'eau 51 formées par des ouvertures traversantes circulaires disposées symétriquement par rapport au plan transversal de l'élément 6 de semelle. Elles alimentent en fluide la semelle et le haut de culasse afférent et communiquent avec un circuit global 52 de distribution du fluide de refroidissement du moteur.

Les deux arrivées d'eau 51 se trouvent à chaque extrémité du lit de rivière 53 représenté en volume sur les figures. Ce lit est sensiblement symétrique par rapport au plan transversal, mais peut être différent au niveau des arrivées de fluide en fonction des calculs et des répartitions thermiques.

Plus précisément le lit 53 de rivière comprend deux branches 54 horizontales qui tournent autour des ouvertures 55 de soupapes, démarrant par un ilot 56 en forme de haricot ouvert vers le haut puis forment une sorte de U écrasé sur son fond 57.

Le lit définit ainsi un parcours d'une largeur moyenne 1 correspondant à la moyenne des distances entre parois interne et externe du lit, et qui est dimensionné avec sa profondeur pour passer les débits de refroidissement avec les vitesses recherchées pour évacuer les calories nécessaires à un bon refroidissement.

Comme indiqué ci-avant la rivière comprend également des étranglements disposés aux emplacements plus générateurs de chaleur pour provoquer une accélération locale entrainant un meilleur refroidissement. La profondeur du lit et non uniquement sa largeur va également pouvoir être modifiée de façon similaire.

Le lit de rivière 53 comprend également des conduits 58 qui rayonnent depuis la zone centrale de l'élément de semelle 6 et sont d'un diamètre par exemple compris entre 1 cm et 0, 2 mm.

Ils passent au travers de l'épaisseur intersiège de deux sièges de soupape contigus puis débouchent orthogonalement sur la face supérieure 7 de l'élément 6 de la semelle.

L'ouverture débouchante est située proche de la première ouverture de pièce de fixation.

Les conduits sont percés et/ou usinés dans la masse de la semelle.

Le circuit de refroidissement 53 comporte également des canaux 59 (par exemple trois) sensiblement perpendiculaires ou formant un angle de l'ordre de 70° avec le plan de la semelle, issus des branches 54 et remontant en rideau le long d'un côté (externe) des canaux d'échappement (non représenté) pour assurer plus particulièrement leur refroidissement.

Des canaux internes (entre les échappements) sont également prévus, tout comme un canal 61 dédié aux canaux d'admission naturellement moins chauds.

Les dimensions et le nombre de ces canaux ménagés dans le haut de culasse sont calculés de façon connue en elle-même par le technicien des moteurs.

On a représenté avec des zones grisées 62 les zones du circuit interne particulièrement chaudes et qui sont donc traitées en conséquences.

Les canaux 58 rayonnant à partir d'une zone centrale de la semelle comportent une extrémité de fourreau d'injecteur, cette dernière étant ainsi très refroidie.

Une fois le refroidissement de l'élément de semelle et des zones adjacentes du haut de culasse (notamment au niveau de l'évacuation des gaz) effectués, le fluide est récupéré par des collecteurs primaires 63, 64 plus haut dans le haut de culasse, eux-mêmes raccordés (liaison 65), à un collecteur appartenant au circuit global 52, de façon connue.

Enfin il est également prévu un chemisage 66 des guides de soupapes et un chemisage ou manchon 67 du fourreau d'injecteur, également raccordés aux collecteurs, éventuellement en étant intercalés entre lesdits canaux et les collecteurs.

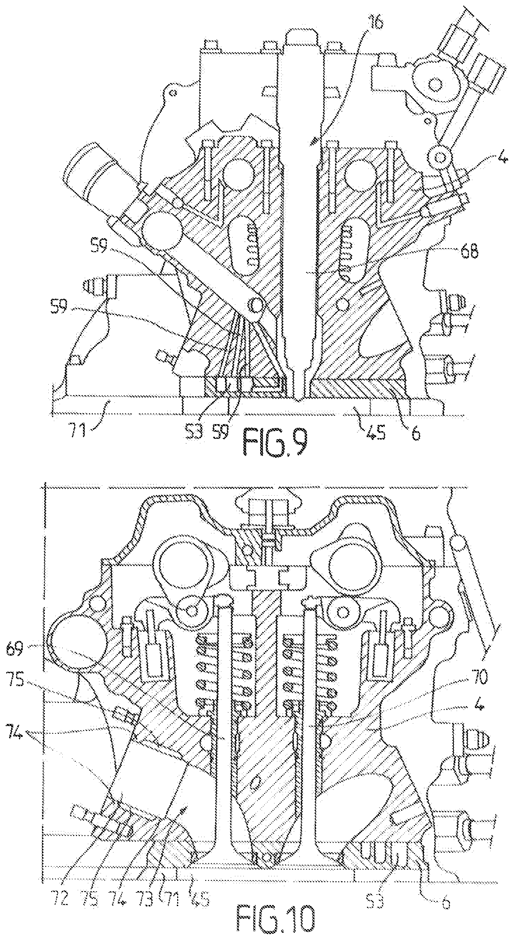

On a représenté sur les figures 9 et 10 un ensemble selon le mode de réalisation de l'invention plus particulièrement décrit ici, avec le haut de culasse 4 en coupe respectivement au niveau de la pièce de fixation 16 avec un injecteur 68, et au niveau d'une soupape 69 d'échappement (et d'une soupape d'admission 70). La culasse 4 et la semelle 6 sont fixées sur le bloc moteur 71 et il est ici prévu un manchon 72 isolant sur le conduit 73 d'échappement des gaz.

Le manchon est par exemple en matière ayant une conductivité thermique très faible. Il est bloqué en position vis à vis du conduit 73 par des portions périphériques 74 à son extrémité ménageant entre elles un espace 75 entre le manchon et la portion de conduit d'échappement en vis-à-vis du manchon, limitant encore les échanges thermiques.

On va maintenant décrire le fonctionnement de la culasse, de la semelle et de l'ensemble, en références aux figures 3 et 8.

Tout au long des cycles moteurs, la chambre de combustion compresse le mélange carburant/comburant et les fait exploser dans la chambre. Ceci est source de chaleur, de température et de contraintes mécaniques transmises aux différents éléments mécaniques du moteur dont la semelle formant face feu qui par ses caractéristiques physiques permet de supporter de plus fortes pressions et températures que les moteurs de l'art antérieur à poids équivalents ou réduits. En particulier lors de la combustion, les pressions atteintes étant très élevées (> 250 bars), la plaque des éléments de semelle se déforme.

Le point sensiblement central d'appui, constitué par le fourreau d'injecteur, crée un point de moindre déformation et réduit de ce fait l'amplitude desdites déformations.

La semelle ou l'élément de semelle est ainsi mieux tenu mécaniquement et permet une meilleure étanchéisation des cylindres de combustion.

La circulation de fluide caloporteur comme décrit en référence à la figure 3 réduit par ailleurs l'effet thermique sur la semelle tout comme au niveau des conduits d'échappement des gaz

Le manchon prévu permet également de limiter la transmission de température au corps de culasse au travers des conduits d'échappement.

En particulier en limitant le transfert de chaleur à l'eau de refroidissement, le manchon permet de réduire la taille nécessaire des échangeurs thermiques comme les radiateurs. De plus les matériaux constitutifs de la culasse sont ainsi maintenus dans des limites de températures correspondant à une plage de meilleures caractéristiques mécaniques.

En outre l'utilisation d'un manchon permet de conserver la chaleur dans les gaz d'échappements ce qui permet un gain de rendement dans les moteurs à turbocompresseur.

Il en résulte l'excellente performance des moteurs quel que soit leur cycle, lorsqu'ils sont équipés de culasse selon l'invention.

Comme il va de soi et comme il résulte également de ce qui précède, la présente invention n'est pas limitée aux modes de réalisation plus particulièrement décrits. Elle en embrasse au contraire toutes les variantes et notamment celles où les vis de fixation périphériques sont plus nombreuses et/ou celle ou la semelle est d'une pièce et compte une ou plusieurs pièces de fixation quelles que soient les dimensions du moteur.

## Revendications

1. Culasse (1) de moteur à piston s'étendant longitudinalement comprenant un corps (4) de culasse et une semelle (5) en au moins un élément (6), la semelle (5) étant à interposer entre une chambre de combustion et le corps (4) de culasse, comportant une face (7) supérieure en vis-à-vis du corps (4) de culasse et une face (8) inférieure opposée à disposer face à la chambre de combustion et étant rapportée sur le corps (4) de culasse, dans laquelle la semelle (5) et le corps (4) sont assemblés par des vis (12) de fixation de la semelle (5) sur la culasse (4) situées vers la périphérie de la semelle (5) et du corps (4) et par au moins une pièce (16) de fixation de la culasse (4) sur la semelle (5) comprenant une extrémité (17) filetée de fixation dans un orifice (18) correspondant de la semelle (5), ledit orifice (18) étant situé plus proche du centre de la semelle (5) que les vis (12) de fixation, **caractérisée en ce que** le corps (4) de culasse est en un matériau d'une masse volumique et d'une dureté plus faibles que celles du matériau constitutif de la semelle (5) et **en ce que** l'effort de serrage de la ou des pièces de fixation est supérieur ou égal à deux fois l'effort de serrage des vis de fixation de la semelle.

2. Culasse selon la revendication 1, **caractérisée en ce que** le corps de culasse est en un matériau d'une conductivité thermique plus forte que celle du matériau constitutif de la semelle.

3. Culasse selon l'une quelconque des revendications précédentes, **caractérisée en ce que** le corps de culasse est en aluminium ou en alliage d'aluminium, en magnésium, ou en alliage de magnésium et la semelle est en acier ou en alliage de nickel.

4. Culasse selon l'une quelconque des revendications précédentes, l'orifice (18) est situé ou sensiblement situé dans un plan longitudinal centré et orthogonal par rapport à la semelle (5).

5. Culasse selon l'une quelconque des revendications précédentes, **caractérisée en ce que** la semelle (5) comprend au moins deux éléments (6) juxtaposés identiques coplanaires comprenant chacun quatre vis (12) de fixation et un orifice (18) .

6. Culasse selon l'une des revendications 4 et 5 dépendante de la 4, **caractérisée en ce que** l'orifice (18) est centré par rapport à la semelle (5) et/ou l'élément (6) de semelle (5).

7. Culasse selon l'une quelconque des revendications précédentes, **caractérisée en ce que** l'orifice (18) est traversant de la semelle, et **en ce que** la pièce (16) de fixation comprend un fourreau agencé pour recevoir individuellement un premier dispositif fonctionnel utile au fonctionnement du moteur et devant interagir avec la chambre de combustion.

8. Culasse selon l'une quelconque des revendications précédentes **caractérisée en ce que**,
la ou les pièces (16) de fixation de la culasse (1) passent à travers des orifices (18) qui traversent le corps (4) de part en part, prennent appui sur le corps (4) et sont vissées dans la semelle (5),
et les vis (12) de fixation de la semelle (5) prennent appui sur la semelle (5) et sont vissées dans le corps (4).

9. Culasse selon l'une quelconque des revendications précédentes, **caractérisée en ce que** la semelle (5) ou chaque élément (6) de semelle (5) s'étendant globalement dans un plan, comprend un circuit (50) interne de refroidissement par circulation d'un fluide caloporteur, ledit circuit (50) comprenant au moins une arrivée (51) de fluide, un lit (53) de rivière de circulation du fluide dans son épaisseur et plusieurs canaux (59) d'évacuation du fluide dans le corps (4) de la culasse, les canaux (59) formant un angle supérieur à 20° par rapport au plan de la semelle (5) en passant autour des conduits (73) d'échappement des gaz du corps (4) de culasse.

10. Culasse selon la revendication 9, **caractérisée en ce que** le lit (53) de rivière, s'étendant suivant un parcours, est d'une largeur moyenne l inférieure à 20% de la dimension transversale de la semelle (5) et comprend le long dudit parcours au moins un étranglement (25) allongé sur au moins 2,5% de la longueur du parcours et la largeur moyenne de l'étranglement (25) parallèlement au plan de la semelle (5) est inférieure à 10% de la largeur de la semelle (5).

11. Culasse selon l'une quelconque des revendications précédentes, **caractérisée en ce que** la semelle (5) comporte au moins un siège de soupape d'une seule pièce avec la semelle.

12. Culasse selon l'une quelconque des revendications précédentes, **caractérisée en ce que** le circuit (53) interne de la semelle (5) présente des perçages (58) intersièges de soupape.

13. Culasse selon l'une quelconque des revendications précédentes, **caractérisée en ce que** la culasse (1) comprend au moins un joint (30 ; 31) d'étanchéité entre le corps (4) et la semelle (5).

14. Culasse selon l'une quelconque des revendications précédentes, **caractérisée en ce que** le corps (4) de culasse comprend des conduits d'admission (32) et d'échappement (73), le conduit d'échappement (73) étant muni d'un manchon (72) ayant une conductivité thermique inférieure à 70 W.m⁻¹.K⁻¹, ledit manchon (72) étant bloqué en position vis-à-vis du conduit (73) d'échappement par des portions (74) d'extrémités à l'écart desquelles un espace (75) existe entre ledit manchon (72) et la portion de conduit (73) d'échappement en vis-à-vis du manchon, pour limiter les échanges thermiques.

15. Culasse selon l'une quelconque des revendications précédentes, **caractérisée en ce que** la semelle (5) comporte une ouverture de passage d'un second dispositif fonctionnel utile au fonctionnement du moteur et devant interagir avec la chambre de combustion.

16. Ensemble comprenant une culasse selon l'une quelconque des revendications 7 et 8 à 15 dépendantes de la 7, **caractérisé en ce que** il comporte des injecteurs de carburant, des bougies ou des dispositifs d'allumage, montés dans les fourreaux en tant que pièces fonctionnelles.

## Patentansprüche

1. Längsgerichteter Kolbenmotor-Zylinderkopf (1), umfassend einen Zylinderkopf-Körper (4) und eine Basis (5) in mindestens einem Element (6), **wobei** die Basis (5) zwischen eine Verbrennungskammer und dem Zylinderkopf-Körper (4) anzuordnen ist, aufweisend eine dem Zylinderkopf-Körper (4) gegenüberliegende obere Seite (7) und eine entgegengesetzte untere Seite (8), die gegenüber der Verbrennungskammer anzuordnen ist und am Zylinderkopf-Körper (4) angebracht ist, in der die Basis (5) und der Körper (4) mittels in Richtung des Randbereichs der Basis (5) und des Körpers (4) angeordneter Schrauben (12) zur Befestigung der Basis (5) an dem Zylinderkopf (4) und mittels mindestens eines Teils (16) zur Befestigung des Zylinderkopfs (4) an der Basis (5) miteinander verbunden sind, welches ein Gewindeende (17) zur Befestigung in einer korrespondierenden Öffnung (18) der Basis (5) umfasst, **wobei** die Öffnung (18) der Mitte der Basis (5) näher liegt als die Befestigungsschrauben (12),
**dadurch gekennzeichnet,**
**dass** der Zylinderkopf-Körper (4) aus einem Werkstoff mit einer Dichte und einer Härte besteht, die geringer sind als diejenigen des Werkstoffs der Basis (5), und dass die Spannkraft des oder der Befestigungsteile größer oder gleich zwei Mal die Spannkraft der Basisnbefestigungsschrauben ist.

2. Zylinderkopf nach Anspruch 1,
**dadurch gekennzeichnet,**
**dass** der Zylinderkopf-Körper aus einem Werkstoff mit einer Wärmeleitfähigkeit besteht, die stärker ist als diejenige des Basisnwerkstoffs.

3. Zylinderkopf nach einem der vorhergehenden Ansprüche,
**dadurch gekennzeichnet,**
**dass** der Zylinderkopf-Körper aus Aluminium oder aus einer Aluminiumlegierung, aus Magnesium oder aus einer Magnesiumlegierung besteht und die Basis aus Stahl oder aus einer Nickellegierung besteht.

4. Zylinderkopf nach einem der vorhergehenden Ansprüche, **wobei** die Öffnung (18) in einer in Bezug auf die Basis (5) orthogonalen und zentrierten Längsebene liegt oder im Wesentlichen liegt.

5. Zylinderkopf nach einem der vorhergehenden Ansprüche,
**dadurch gekennzeichnet,**
**dass** die Basis (5) mindestens zwei nebeneinander angeordnete identische koplanare Elemente (6) umfasst, welche jeweils vier Befestigungsschrauben (12) und eine Öffnung (18) umfassen.

6. Zylinderkopf nach einem der Ansprüche 4 und 5 abhängig von Anspruch 4,
**dadurch gekennzeichnet,**
**dass** die Öffnung (18) in Bezug auf die Basis (5) und oder das Element (6) der Basis (5) zentriert ist.

7. Zylinderkopf nach einem der vorhergehenden Ansprüche,
**dadurch gekennzeichnet,**
**dass** die Öffnung (18) durch die Basis durchgeht und dass das Befestigungsteil (16) eine Hülle umfasst, die angeordnet ist, um eine erste für den Betrieb des Motors nützliche funktionale Vorrichtung individuell aufzunehmen, welche mit der Verbrennungskammer interagieren muss.

8. Zylinderkopf nach einem der vorhergehenden Ansprüche,
**dadurch gekennzeichnet,**
**dass** das oder die Teile (16) zur Befestigung des Zylinderkopfes (1) durch durch den gesamten Körper (4) durchgehende Öffnungen (18) hindurchgeführt werden, sich auf dem Körper (4) abstützen und in der Basis (5) verschraubt sind, und
**dass** die Schrauben (12) zur Befestigung der Basis (5) sich auf der Basis (5) abstützen und in dem Körper (4) verschraubt sind.

9. Zylinderkopf nach einem der vorhergehenden Ansprüche,
**dadurch gekennzeichnet,**
**dass** die Basis (5) oder jedes sich insgesamt in einer Ebene erstreckende Element (6) der Basis (5) einen internen Kreislauf (50) zur Kühlung durch Zirkulation eines Wärmeträgermediums umfasst, **wobei** der Kreislauf (50) mindestens eine Mediumzufuhr (51), ein Flussbett (53) für die Zirkulation des Mediums in seiner Dicke und mehrere Kanäle (59) für die Ableitung des Mediums in dem Zylinderkopf-Körper (4) umfasst, wobei die Kanäle (59) einen Winkel mit der Ebene der Basis (5) bilden, welcher größer als 20° ist, und dabei um die Gasautrittsleitungen (73) des Zylinderkopf-Körpers (4) laufen.

10. Zylinderkopf nach Anspruch 9,
**dadurch gekennzeichnet,**
**dass** das sich gemäß einer Strecke erstreckende Flussbett (53) eine Durchschnittsbreite l aufweist, die geringer ist als 20% der Querabmessung der Basis (5) ist, und entlang der Strecke mindestens eine Verengung (25) umfasst, die sich auf mindestens et 2,5% der Länge der Strecke erstreckt, und die Durchschnittsbreite der Verengung (25) parallel zur Ebene der Basis (5) geringer ist als 10% der Breite der Basis (5).

11. Zylinderkopf nach einem der vorhergehenden Ansprüche,
**dadurch gekennzeichnet,**
**dass** die Basis (5) mindestens ein Ventilsitz aufweist, der einstückig mit der Basis ausgebildet ist.

12. Zylinderkopf nach einem der vorhergehenden Ansprüche,
**dadurch gekennzeichnet,**
**dass** der interne Kreislauf (53) der Basis (5) ventilsitzübergreifende Bohrungen (58) aufweist.

13. Zylinderkopf nach einem der vorhergehenden Ansprüche,
**dadurch gekennzeichnet,**
**dass** der Zylinderkopf (1) mindestens eine Dichtung (30 ; 31) zwischen dem Körper (4) und der Basis (5) umfasst.

14. Zylinderkopf nach einem der vorhergehenden Ansprüche,
**dadurch gekennzeichnet,**
**dass** der Zylinderkopf-Körper (4) Einlass- (32) und Auslassleitungen (73) umfasst, wobei die Auslassleitung (73) mit einer Hülse (72) mit einer Wärmeleitfähigkeit versehen ist, die geringer ist als 70 W.m-1.K-1, **wobei** die Hülse (72) gegenüber der Auslassleitung (73) durch Endabschnitte (74) arretiert ist, abseits deren sich ein Raum (75) zwischen der Hülse (72) und dem Abschnitt der Auslassleitung (73) gegenüber der Hülse befindet, um den Wärmeaustausch zu begrenzen.

15. Zylinderkopf nach einem der vorhergehenden Ansprüche,
**dadurch gekennzeichnet,**
**dass** die Basis (5) eine Öffnung für die Durchführung einer für den Betrieb des Motors nützlichen zweiten funktionalen Vorrichtung aufweist, die mit der Verbrennungskammer interagieren muss.

16. Anordnung mit einem Zylinderkopf nach einem der Ansprüche 7 und 8 bis 15 abhängig von Anspruch 7,
**dadurch gekennzeichnet,**
**dass** sie Kraftstoffeinspritzvorrichtungen, Kerzen oder Zündvorrichtungen aufweist, die in den Hüllen als Funktionsteile montiert sind.

## Claims

1. Cylinder head (1) of piston engine extending longitudinally including a cylinder head body (4) and a flange (5) in at least one element (6), the flange (5) to be interposed between a combustion chamber and the cylinder head body (4), comprising an upper face (7) opposite the cylinder head body (4) and an opposed lower face (8) to be disposed facing the combustion chamber and being mounted on the cylinder head body (4), in which the flange (5) and the body (4) are assembled by bolts (12) for fixing the flange (5) to the cylinder head (4), which are situated towards the periphery of the flange (5) and body (4), and by at least one component (16) for fixing the cylinder head (4) to the flange (5) including one threaded end (17) for fixing in a corresponding orifice (18) in the flange (5), said orifice (18) being located closer to the centre of the flange (5) than the fixing bolts (12), **characterised in that** the cylinder head body (4) is made of a material having a volumetric mass and hardness lower than those of the material that constitutes the flange (5) and **in that** the clamping force of the fixing component or components is greater than or equal to twice the clamping force of the flange fixing bolts.

2. Cylinder head according to claim 1, **characterised in that** the cylinder head body is made of a material having a thermal conductivity greater than that of the material that constitutes the flange

3. Cylinder head according to any one of the preceding claims, **characterised in that** the cylinder head body is made of aluminium or aluminium alloy, magnesium or magnesium alloy, and the flange is made of steel or nickel alloy.

4. Cylinder head according to any one of the preceding claims, **characterised in that** the orifice (18) is located or substantially situated in a longitudinal plane centred on and perpendicular to the flange (5).

5. Cylinder head according to any one of the preceding claims, **characterised in that** the flange (5) includes at least two identical juxtaposed coplanar elements (6) each including four fixing bolts (12) and an orifice (18).

6. Cylinder head according to one of claims 4 and 5 which is dependent on 4, **characterised in that** the orifice (18) is centred relative to the flange (5) and/or the flange element (6).

7. Cylinder head according to any one of the preceding claims, **characterised in that** the orifice (18) passes through the flange, and **in that** the fixing component (16) includes a sheath arranged to receive individually a first functional device useful for operation of the engine, which is to interact with the combustion chamber.

8. Cylinder head according to any one of the preceding claims, **characterised in that** the component or components (16) for fixing the cylinder head (1) pass through orifices (18) which traverse the body (4) from one side to the other, are supported on the body (4) and are screwed into the flange (5), and the bolts (12) for fixing the flange (5) are supported on the flange (5) and are screwed into the body (4).

9. Cylinder head according to any one of the preceding claims, **characterised in that** the flange (5) or each flange element (6) extending overall in one plane, includes a cooling internal circuit (50) to circulate a heat-carrying fluid, said circuit (50) including at least one fluid inlet (51), a channel (53) for circulation of the fluid across its thickness and a plurality of channels (59) for discharge of the fluid into the body (4) of the cylinder head, the channels (59) forming an angle greater than 20° relative to the plane of the flange (5) while passing around the exhaust conduits (73) for the gases from the cylinder head body (4) .

10. Cylinder head according to claim 9, **characterised in that** the channel (53), extending along a path, has an average width l less than 20% of the transverse dimension of the flange (5) and includes along said path at least one elongate constriction (25) over at least 2.5% of the length of the path, and the average width of the constriction (25) parallel to the plane of the flange (5) is less than 10% of the width of the flange (5).

11. Cylinder head according to any one of the preceding claims, **characterised in that** the flange (5) comprises at least one valve seat in one piece with the flange.

12. Cylinder head according to any one of the preceding claims, **characterised in that** the internal circuit (53) of the flange (5) has bores (58) between valve seats.

13. Cylinder head according to any one of the preceding claims, **characterised in that** the cylinder head (1) includes at least one sealing gasket (30; 31) between the body (4) and the flange (5).

14. Cylinder head according to any one of the preceding claims, **characterised in that** the cylinder head body (4) includes admission (32) and exhaust (73) conduits, the exhaust conduit (73) being provided with a sleeve (72) having a thermal conductivity less than 70 W.m-1.K-1, said sleeve (72) being locked in position in relation to the exhaust conduit (73) by end portions (74) at a distance from which a space (75) exists between said sleeve (72) and the portion of the exhaust conduit (73) facing the sleeve, to limit heat exchanges.

15. Cylinder head according to any one of the preceding claims, **characterised in that** the flange (5) comprises an opening for passage of a second functional device useful for operation of the engine, to interact with the combustion chamber.

16. Assembly including a cylinder head according to any one of claims 7 and 8 to 15 which are dependent on 7, **characterised in that** it comprises fuel injectors, spark plugs or ignition devices, mounted in the sheaths as functional components.
